# EUROPEAN PATENT APPLICATION

(11) **EP 4 130 744 A1**
(43) Date of publication of application: **08.02.2023**
(21) Application number: 21776863.9
(22) Date of filing: 25.03.2021
(51) Int. Cl.: G01N 33/543

(54) **IMMUNOCHROMATOGRAPHY KIT AND IMMUNOCHROMATOGRAPHY METHOD**

(30) Priority: 26.03.2020 JP 2020056216
(71) Applicant: FUJIFILM Corporation, Tokyo 106-8620 (JP)
(72) Inventor: YAMAMOTO Ko, Ashigarakami-gun, Kanagawa 258-8538 (JP); WADA Atsuhiko, Ashigarakami-gun, Kanagawa 258-8538 (JP)
(74) Representative: Owen, Deborah Jane
(86) International application number: PCT/JP2021/012448
(87) International publication number: WO 2021/193792

(57) **Abstract**

An object of the present invention is to provide a chromatographic kit and a chromatographic method which have high-sensitivity and in which occurrence of false positives is inhibited. According to the present invention, an immunochromatographic kit is provided, the immunochromatographic kit including: a label substance modified with a first antibody having binding properties with respect to a test substance; a porous carrier having a reaction site holding a second antibody having binding properties with respect to the test substance; an amplification reagent; and a third antibody having an immune host which is the same as that of at least one antibody of the first antibody or the second antibody and not recognizing the test substance.

## Description

### Technical Field

The present invention relates to an immunochromatographic kit including a label substance, a porous carrier, and an amplification reagent. The present invention further relates to an immunochromatographic method using a label substance, a porous carrier, and an amplification reagent.

### Background Art

Among immunoassay methods, an immunochromatographic method is generally utilized, because operation is easy and measurement can be performed within a short period of time. Competitive responses or sandwich-based responses are widely used as an immune response used in the immunochromatographic method. Among them, sandwich-based responses are the mainstream in the immunochromatographic method, and in a typical example thereof, the following operation is performed in order to detect a test substance composed of antigens in a specimen. First, fine particles sensitized with an antibody against an antigen which is a test substance are immobilized as solid-phase fine particles on an immunochromatographic carrier, or an antibody itself is directly immobilized on an immunochromatographic carrier, and thereby a chromatographic carrier having a reaction site is prepared. Meanwhile, labeled fine particles are sensitized with an antibody against a test substance to prepare sensitized labeled fine particles. These sensitized labeled fine particles are then immunochromatographically moved together with a specimen on the immunochromatographic carrier. By the above operation, the immobilized antibody serves as an immobilization reagent at the reaction site formed on the immunochromatographic carrier, and the sensitized labeled fine particles specifically bind to this immobilized antibody via an antigen which is a test substance. As a result, by visually determining the presence or absence or a degree of signals generated by the sensitized labeled fine particles trapped at the reaction site, it is possible to measure the presence or absence or an amount of the test substance in a specimen.

Patent Document 1 discloses a test kit for an immunochromatographic analysis to be used for an immunochromatographic analysis, and discloses that false positives at the time of detection are inhibited by using an immunoglobulin.

Patent Document 2 discloses an immunochromatographic method for detecting a test substance by sensitization using a compound containing silver, and a reducing agent for silver ions. The above-mentioned silver amplification method has an advantage that high-sensitivity can be achieved.

On the other hand, Patent Document 3 discloses a method for measuring a test substance using a mixed solution containing first dry particles which are modified with a first antibody having binding properties specific to a test substance and which have a label, second dry particles which are modified with a second antibody not having binding properties specific to the test substance and do not have a label, and the test substance.

### Prior Art Documents

### Patent Documents

Patent Document 1: JP4514233B
Patent Document 2: JP4892500A
Patent Document 3: JP2015-72249A

### SUMMARY OF THE INVENTION

In the silver amplification method, there is a problem that a signal for silver amplification is expressed even in the case of non-specific adsorption at the level that does not cause a problem in a general immunochromatographic product on which silver amplification is not performed, which results in a false positive diagnosis. In particular, non-specific adsorption is likely to occur in samples containing various components (blood and the like), and countermeasures are required. As a method for inhibiting false positives, it is conceivable to change the formulation of a cleaning agent after adjusting a pH, but there is a drawback of resulting in a trade-off between specificity and sensitivity.

An object of aspects of the present invention is to provide an immunochromatographic kit and an immunochromatographic method which have high-sensitivity and in which occurrence of false positives is inhibited.

As a result of intensive studies to achieve the above-mentioned object, the inventors of the present invention have found that measurement can be performed with high-sensitivity while inhibiting occurrence of false positives by adding particles modified with an antibody not having binding properties specific to a test substance to a sample diluent solution to be spotted on a kit, and amplifying a signal by further using an amplification reagent, thereby completing the present invention.

That is, according to aspects of the present invention, the following inventions are provided.
[1] An immunochromatographic kit comprising:
   a label substance modified with a first antibody having binding properties with respect to a test substance;
   a porous carrier having a reaction site holding a second antibody having binding properties with respect to the test substance;
   an amplification reagent; and
   a third antibody having an immune host which is the same as that of at least one antibody of the first antibody or the second antibody and not recognizing the test substance.
[2] The immunochromatographic kit according to [1], in which the porous carrier further has a fourth antibody having binding properties with respect to the first antibody.
[3] The immunochromatographic kit according to [1] or [2], in which the third antibody is used at a concentration of 0.0096 mg/mL or more.
[4] The immunochromatographic kit according to any one of [1] to [3], in which at least one of the first antibody or the second antibody is F(ab')₂, Fab, Fab', or Fv.
[5] The immunochromatographic kit according to any one of [1] to [4], in which the label substance is a metal colloid.
[6] The immunochromatographic kit according to any one of [1] to [5], in which the amplification reagent contains a reducing agent for silver ions, and a compound containing silver.
[7] The immunochromatographic kit according to [6], in which the reducing agent for silver ions contains divalent iron ions.
[8] The immunochromatographic kit according to any one of [1] to [7], in which the immunochromatographic kit is used in measurement of the test substance in whole blood, serum, or plasma.
[9] An immunochromatographic method comprising:
   a step of spreading, on a porous carrier having a reaction site holding a second antibody having binding properties with respect to a test substance, the test substance, a label substance modified with a first antibody having binding properties with respect to the test substance, and a third antibody having an immune host which is the same as that of at least one antibody of the first antibody or the second antibody and not recognizing the test substance, in a state in which a composite body of the test substance and the label substance is formed;
   a step of trapping the composite body at the reaction site; and
   a step of amplifying a signal of the label substance with an amplification reagent to detect the test substance.
[10] The immunochromatographic method according to [9], in which the test substance is a test substance in whole blood, serum, or plasma.

According to a chromatographic kit and a chromatographic method of the aspects of the present invention, measurement can be performed with high-sensitivity while inhibiting the occurrence of false positives.

### BRIEF DESCRIPTION OF THE DRAWINGS

Fig. 1 is a perspective view showing an example of a chromatographic kit.
Fig. 2 is an exploded schematic perspective view showing the example of the chromatographic kit.
Fig. 3 is a schematic side view showing a positional relationship between an inspection strip, and a first pot and a second pot.

### DESCRIPTION OF THE PREFERRED EMBODIMENTS

Hereinafter, embodiments of the present invention will be described in detail.

Numerical value ranges expressed using "to" in the present specification mean a range including numerical values described before and after "to" as a minimum value and a maximum value.

An immunochromatographic kit of the embodiment of the present invention is an immunochromatographic kit including: a label substance modified with a first antibody having binding properties with respect to a test substance; a porous carrier having a reaction site holding a second antibody having binding properties with respect to the test substance; an amplification reagent; and a third antibody having an immune host which is the same as that of at least one antibody of the first antibody or the second antibody and not recognizing the test substance.

An immunochromatographic method according to the embodiment of the present invention is a method including:
a step of spreading, on a porous carrier having a reaction site holding a second antibody having binding properties with respect to a test substance, the test substance, a label substance modified with a first antibody having binding properties with respect to the test substance, and a third antibody having an immune host which is the same as that of at least one antibody of the first antibody or the second antibody and not recognizing the test substance, in a state in which a composite body of the test substance and the label substance is formed;
a step of trapping the composite body at the reaction site; and
a step of amplifying a signal of the label substance with an amplification reagent to detect the test substance.

In the present invention, the third antibody having an immune host which is the same as that of at least one antibody of the first antibody or the second antibody and not recognizing the test substance is used. The third antibody can inhibit non-specific adsorption by reacting with a substance (substance that is not desired to be detected) which cross-reacts with the first antibody or the second antibody.

The mechanism for inhibiting false positives in the present invention is presumed as follows.

False positives occur in a case where non-specific adsorption substances (for example, Human Anti-Mouse Antibody (HAMA) and the like) in human serum are non-specifically adsorbed to the antibody of a membrane and the antibody of a gold colloid. In a correct antigen-antibody reaction, the antibody of the membrane and the antibody that modifies a gold colloid are adsorbed to a target antigen, respectively, and a signal is expressed at the reaction site (detection line). On the other hand, in a reaction by non-specific adsorption components in a sample (blood and the like), a signal is expressed at the reaction site (detection line) even though a non-specific component is adsorbed instead of a target antigen, and a target antigen is not detected. In the present invention, in order to inhibit false positives due to non-specific adsorption substances, an antibody (the above-mentioned third antibody) of the same type as the antibody used in the immunochromatographic kit is modified into particles as a dummy antibody, and reacted in a pre-stage of the antigen-antibody reaction. By the dummy antibody reacting with the non-specific adsorption substance in the sample without reacting with the antigen, a non-specific reaction with an NS 1 protein which is a target antigen is inhibited, thereby inhibiting false positives. According to the present invention, the reaction with a non-specific reaction substance in serum is inhibited, which makes it possible to more accurately determine detection of a target antigen.

As described above, the characteristic of the present invention is that a dummy antibody (antibody having the same immune host as that of at least one antibody of the first antibody having binding properties with respect to the test substance or the second antibody having binding properties with respect to the test substance) is added to the kit, which makes it possible to inhibit false positives in the silver-amplified immunochromatographic kit. In particular, the present invention enables to embody the reduction of false positives in fragmented antibodies. This indicates that the false positive inhibitory effect is significant even for non-specific adsorption which has not been solved in the related art.

### 1. Immunochromatography

In general, an immunochromatographic method is a technique of simply, rapidly, and specifically determining and measuring a test substance by the following technique. That is, a membrane (porous carrier) is used as an immobilized phase, where the membrane may have a label substance-trapping region having at least one reaction site having the second antibody having binding properties with respect to the test substance. On this porous carrier, a liquid containing the label substance modified with the first antibody having binding properties with respect to the test substance is moved chromatographically as a moving layer to reach the label substance-trapping region having the reaction site while the test substance and the label substance specifically bind to each other. The technique is a technique of qualitatively and quantitatively analyzing the presence of the test substance in a test specimen visually or using an appropriate device by utilizing that, in the reaction site of the label substance-trapping region, a composite body of the test substance and the label substance specifically binds to the immobilized second antibody, and thereby the label substance is concentrated in the second antibody only in a case in which the test substance is present in the test specimen.

In the immunochromatographic method of the embodiment of the present invention, using two kinds of amplification reagents used for amplifying signals of the label substance, specifically, the compound containing silver and the reducing agent for silver ions, it is possible to amplify signals by an amplification reaction using, as a nucleus, the composite body of the label substance and the test substance bound to an immobilization reagent on the label substance-trapping region, and as a result, it is possible to achieve high-sensitivity. According to the present invention, rapid chromatography with high-sensitivity can be performed.

### 2. Specimen containing test substance (also called test specimen)

A test specimen that can be analyzed using the chromatographic method and the kit of the embodiment of the present invention is not particularly limited as long as it is a specimen that may contain a test substance. Examples thereof include biological specimens, particularly body fluids (for example, whole blood, serum, plasma, spinal fluid, tears, sweat, urine, pus, nasal discharge, nasal swab, pharyngeal swab, nasal aspirate, or sputum) of animals (particularly humans), or excrements (for example, feces), organs, tissues, mucous membranes and skin, scraped samples (swabs) that contain these substances, or animals and plants themselves or dried substances thereof. The test substance is preferably a test substance in whole blood, serum, or plasma. That is, the specimen containing the test substance is preferably whole blood, serum, or plasma.

It is possible to use the test specimen as it is, or in a form of an extraction liquid obtained by extracting the test specimen using an appropriate solvent for extraction, in a form of a diluent solution obtained by diluting an extraction liquid with an appropriate diluent, or in a form of in which an extraction liquid has been concentrated by an appropriate method. As the solvent for extraction used in the present invention, it is possible to use a solvent used in a general immunological analysis method (for example, water, physiological saline, a buffer solution, and the like), or a water-miscible organic solvent that enables performing of a direct antigen-antibody reaction by diluting with such a solvent.

### 3. Diluent solution

The diluent solution at the time of spreading the specimen containing the test substance on the porous carrier contains a nonionic surfactant and casein.

Examples of nonionic surfactants include polyoxyethylene alkyl ether, polyoxyethylene/polyoxypropylene alkyl ether, polyoxyethylene sorbitan fatty acid ester (Tween (registered trademark) series), polyoxyethylene p-t-octylphenyl ether (Triton (registered trademark) series), polyoxyethylene p-t-nonylphenyl ether (Triton (registered trademark) N series), alkyl polyglucoside, fatty acid diethanolamide, alkyl monoglyceryl ether, and the like. Among them, polyoxyethylene sorbitan fatty acid ester (Tween (registered trademark) series), which is a polysorbate surfactant, is preferable, and, for example, Tween (registered trademark) 40, Tween (registered trademark) 60, and Tween (registered trademark) 80 are particularly preferable.

The content of the nonionic surfactant in the diluent solution is preferably within the range of 0.01 to 10 mass%, more preferably within the range of 0.05 to 5 mass%, and even more preferably within the range of 0.1 to 1 mass%.

The content of the casein in the diluent solution is preferably within the range of 0.001 to 1 mass%, more preferably within the range of 0.005 to 0.5 mass%, and even more preferably within the range of 0.01 to 0.1 mass%.

### 4. Constitution

In the immunochromatographic kit of the embodiment of the present invention, a chromatographic strip can be incorporated and used. The chromatographic strip that can be used is not particularly limited as long as it is a chromatographic strip that can be used in general immunochromatographic methods.

The chromatographic strip that can be used in the present invention has a label substance-holding region and the label substance-trapping region from the upstream direction to the downstream direction of a spreading direction of a test specimen containing a test substance. In a preferred aspect, the chromatographic strip further has a region (coloring region) containing a coloring reagent. A more preferred aspect of the present invention is an aspect in which the region containing a coloring reagent is located in the downstream direction of the label substance-trapping region. Furthermore, an aspect, in which a specimen-added pad, a label substance-holding pad having a label substance-holding region (for example, gold colloid antibody-holding pad), an antibody-immobilized membrane that is a porous carrier (for example, an antibody-immobilized membrane having a label substance-trapping region), and a water absorption pad are disposed in this order on a pressure-sensitive adhesion sheet, is preferably used. The antibody-immobilized membrane that is a porous carrier has the label substance-trapping region that is a region having at least one reaction site on which an antibody that specifically binds to a test substance is immobilized, or as desired, the antibody-immobilized membrane may further have a control site (sometimes referred to as a control region) which is a region in which an antibody for control or an antigen is immobilized.

The label substance-holding pad which has the label substance-holding region and can be used in the present invention can be prepared by preparing a suspension containing the label substance, applying the suspension to an appropriate water absorption pad (for example, glass fiber pad), and then drying the suspension.

### 4-1. Label substance

As the label substance used in the present invention, it is preferable to use a label substance containing a metal as a label used for labeling the first antibody having binding properties with respect to the test substance. The label substance is more preferably a metal particle. As the type of metal that can be used in the present invention, noble metals such as gold, silver, and platinum, iron, lead, copper, cadmium, bismuth, antimony, tin, or mercury can be preferably used, or compounds thereof can be used. Noble metals such as gold, silver, and platinum can be more preferably used. As a preferred form of the label substance containing a metal which can be used in the present invention, it is possible to use a metal colloid or a metal sulfide. In the present invention, as the metal colloid, platinum colloid, gold colloid, silver colloid, iron colloid, aluminum hydroxide colloid, or the like can be preferably used, and as the metal sulfide, each of sulfides of iron, silver, lead, copper, cadmium, bismuth, antimony, tin, or mercury can be preferably used. In the present invention, platinum colloid, gold colloid, or silver colloid can be more preferably used, and gold colloid can be most preferably used. In a case where gold colloid particles are used as the metal colloid, commercially available gold colloid particles may be used. Alternatively, gold colloid particles can be prepared by a conventional method such as a method of reducing chlorauric acid with sodium citrate (Nature Physical Science, 241 (1973) 20, and the like).

An average particle size of the metal colloid is preferably about 1 nm to 500 nm, more preferably 3 to 100 nm, and particularly preferably 5 to 60 nm. An average particle size of the metal colloid used in the present invention can be measured with a commercially available particle size distribution meter or the like. As a method of measuring particle size distribution, optical microscopy, confocal laser microscopy, electron microscopy, atomic force microscopy, a static light scattering method, a laser diffraction method, a dynamic light scattering method, a centrifugal sedimentation method, an electric pulse measurement method, a chromatographic method, an ultrasonic attenuation method, and the like are known, and devices corresponding to the respective principles are commercially available.

As a method of measuring an average particle size, a dynamic light scattering method can be preferably used because of a particle size range and ease of measurement. Examples of commercially available measuring devices using dynamic light scattering include NANOTRAC UPA (Nikkiso Co., Ltd.), a dynamic light scattering type particle size distribution measuring device LB-550 (HORIBA, Ltd.), a concentrated particle size analyzer FPAR-1000 (Otsuka Electronics Co., Ltd.), and the like. In the present invention, an average particle size is obtained as a value of a median diameter (d = 50) measured at a measurement temperature of 25°C.

According to the present invention, in chromatography using a metal colloid label or metal sulfide label, another metal alloy label (hereinafter sometimes referred to as a metallic label), or a metal-containing polymer particle label as a label substance for detection, signals of the metallic label can be amplified. Specifically, in a case where, after formation of a composite body of a test substance and a label for detection, silver ions supplied from a compound containing silver such as an inorganic silver salt or an organic silver salt are brought into contact with a reducing agent for silver ions, the silver ions are reduced by the reducing agent, and thereby silver particles are generated, the silver particles are deposited on a metallic label with the metallic label as a nucleus, and therefore, the metallic label is amplified, and analysis of the test substance can be performed with high-sensitivity. That is, in the chromatographic method of the embodiment of the present invention, the silver particles generated by the reducing action of silver ions by the reducing agent are used to perform a reaction for depositing on a label of an immune composite body, and signals thus amplified are analyzed.

### 4-2. First antibody and second antibody

In the present invention, the label substance is modified with the first antibody having binding properties with respect to the test substance. The first antibody is an antibody against the test substance (antigen), and has binding properties with respect to the test substance.

The immunochromatographic kit of the embodiment of the present invention includes the second antibody, which has binding properties with respect to the test substance, in the label substance-trapping region. The second antibody is an antibody against the test substance (antigen), and has binding properties with respect to the test substance.

The antibody (that is, the first antibody and the second antibody) having binding properties with respect to the test substance is not particularly limited. For example, it is possible to use antisera prepared from animal sera immunized with the test substance, immunoglobulin fractions purified from antiserum, monoclonal antibodies obtained by cell fusion using animal spleen cells immunized with the test substance, or fragments thereof [for example, F(ab')₂, Fab, Fab', or Fv]. Preparation of these antibodies can be performed by a conventional method. At least one of the first antibody or the second antibody is preferably F(ab')₂, Fab, Fab', or Fv.

In the present invention, a method of modifying the label substance using the first antibody can be performed according to, for example, a conventionally known method described below (for example, The Journal of Histochemistry and Cytochemistry, 30, 7 (1982) 691-696) in a case of binding a metal colloid and an antibody to each other. As a specific example, a metal colloid and the first antibody are mixed in an appropriate buffer solution at room temperature for 5 minutes or longer. After the reaction, a precipitate obtained by centrifugation is dispersed in a solution containing a dispersant such as polyethylene glycol, and thereby a desired metal colloid-labeled antibody can be obtained.

### 4-3. Third antibody

In the present invention, the third antibody having an immune host which is the same as that of at least one antibody of the first antibody or the second antibody and not recognizing the test substance is used.

As the third antibody, it is possible to use antisera prepared from sera of animals immunized with substances other than the test substance, immunoglobulin fractions purified from antisera, monoclonal antibodies obtained by cell fusion using spleen cells of animals immunized with the test substance, or fragments thereof [for example, F(ab')₂, Fab, Fab', or Fv]. Preparation of these antibodies can be performed by a conventional method. Furthermore, the antibody may be subjected to modification as in the case in which the antibody is a chimeric antibody or the like. Alternatively, a commercially available antibody, or an antibody prepared by a known method from animal sera or culture supernatant can be used.

As the third antibody, one that has been immobilized on particles may be used.

As a method for immobilizing the third antibody on particles, for example, methods disclosed in JP2000-206115A or the protocol attached to FluoSpheres (registered trademark) Polystyrene Microsphere F8813 of Molecular Probe Co., Ltd. can be used. Furthermore, as the principle of immobilizing the antibody on particles, any of the principle of physical adsorption or the principle of chemical bond by covalent bond can be adopted. As a blocking agent that covers the surface of particles not coated with the antibody after the antibody is immobilized on the particles, it is possible to use known substances such as bovine serum albumin (BSA) and skim milk, casein, soybean-derived components, fish-derived components, polyethylene glycol, and commercially available blocking agents for immune responses containing these substances and substances having the same properties as these can be used. These blocking agents can be subjected to pretreatment such as partial denaturation with heat, acid, alkali, or the like, as necessary.

In the immunochromatographic method of the embodiment of the present invention, the third antibody is preferably used at the concentration of 0.0096 mg/mL or more, and the concentration may be 0.01 mg/mL or more, 0.05 mg/mL or more, and 0.1 mg/mL or more, 0.2 mg/mL or more, 0.3 mg/mL or more, or 0.4 mg/mL or more. The upper limit of the concentration of the third antibody is not particularly limited, but it is generally 2.0 mg/mL or less, and may be 1.0 mg/mL or less, or 0.7 mg/mL or less.

### 4-4. Porous carrier

As the porous carrier that can be used in the present invention, a nitrocellulose carrier (such as a nitrocellulose membrane), a cellulose membrane, an acetyl cellulose membrane, a polysulfone membrane, a polyether sulfone membrane, a nylon membrane, a glass fiber, a non-woven fabric, a cloth, a thread, and the like are particularly preferable. The porous carrier is preferably an insoluble carrier.

In the present invention, the label substance-trapping region of the porous carrier has the reaction site on which the second antibody having binding properties with respect to the test substance is immobilized. The second antibody having binding properties with respect to the test substance may be directly immobilized on a part of the porous carrier by a physical or chemical bond, and thereby the reaction site is formed, or the second antibody may physically or chemically bind to fine particles such as latex particles, these fine particles are trapped on a part of the porous carrier and immobilized, and thereby the reaction site is formed. The porous carrier is preferably used after immobilizing the second antibody having binding properties with respect to the test substance thereon and then subjecting the porous carrier to a non-specific adsorption prevention treatment such as a treatment with an inactive protein. The porous carrier can also be preferably used in a form of having a plurality of reaction sites, and as desired, it may further have the above-mentioned control site as a part of the label substance-trapping region. As the control site, a site, which has a fourth antibody having binding properties with respect to the first antibody, and the like can be provided.

### 4-5. Label substance-holding pad

In the present invention, an aspect in which a label substance-holding pad having a label substance-holding region, preferably a gold colloid-holding pad, is incorporated into the chromatographic kit and used is preferable. As a material of the label substance-holding pad, for example, cellulose filter paper, glass fiber, non-woven fabric, and the like can be preferably used, and it is possible to obtain the label substance-holding region by impregnating a certain amount of the label substance prepared as described above and drying it.

### 4-6. Specimen-added pad

It is preferable to use the chromatographic kit of the embodiment of the present invention by further incorporating a specimen-added pad thereto. For the specimen-added pad, an aspect in which the specimen-added pad has not only a function of receiving an added specimen containing a test substance but also has a function of filtering insoluble particles and the like in the specimen is preferable. Examples of materials of the specimen-added pad include materials having uniform characteristics such as cellulose filter paper, glass fibers, polyurethane, polyacetate, cellulose acetate, nylon, and cotton cloth. In addition, in order to prevent the test substance in the specimen from non-specifically adsorbing to a material of the specimen-added pad and lowering accuracy of analysis during analysis, a material forming the specimen-added part can also be used after being treated to a non-specific adsorption prevention treatment in advance. In the present invention, the specimen-added pad may also serve as the label substance-holding pad having the label substance-holding region.

### 4-7. Water absorption pad

In the present invention, it is preferable to use the chromatographic kit by incorporating a water absorption pad thereto. The water absorption pad is a site that physically absorbs an added specimen by chromatographic migration and also absorbs and removes unreacted label substances that are not insolubilized in a detection part of a chromatographic carrier, and water absorbent materials such as cellulose filter paper, non-woven fabric, cloth, and cellulose acetate are used. Because a chromatographic speed after a chromatographic tip end portion of the added specimen reaches the water absorption pad depends on materials, sizes, and the like of the water absorption pad, it is possible to set a speed that is suitable for measurement of the test substance by selecting materials, sizes, and the like of the water absorption pad.

### 5. Coloring reagent for detecting reducing agent for silver ions

In the chromatographic kit used in the present invention, a coloring reagent is preferably carried by a porous carrier.

In the present invention, it is preferable to use, for example, a compound that reacts with ions and develops color as the coloring reagent for detecting the reducing agent for silver ions. Although the amplification reagent will be described later in the present specification, in a case where the second amplification reagent is a reagent containing divalent iron ions (Fe²⁺), as a coloring reagent thereof, a compound that reacts with Fe²⁺ ions and develops color can be used, for example. As the compound that reacts with Fe²⁺ ions and develops color, it is possible to use a compound capable of developing color by forming a complex with Fe²⁺ ions. As specific examples of the compound that reacts with Fe²⁺ ions and develops color, it is possible to use compounds having a phenanthroline skeleton [for example, 1,10-phenanthroline, 5-methyl phenanthroline, 5-nitrophenanthroline, bathophenanthroline (4,7-diphenyl-1,10-phenanthroline), bathophenanthroline disulfate, and the like], or compounds having a bipyridine skeleton [for example, 2,2'-bipyridine and the like], and compounds having a phenanthroline skeleton can be preferably used. In addition, in a case in which a pH of an aqueous solution containing a test specimen and a pH of an aqueous solution containing the second amplification reagent are different from each other, it is possible to preferably use a reagent in which tint is changed because of structural change occurring due to H⁺ ions in order to detect the second amplification reagent. Particularly, in a case in which the aqueous solution containing the second amplification reagent is acidic (where a pH is lower than 7, and a concentration of H⁺ ions is high), as a pH indicator for an acidic region, it is preferable to appropriately select compounds and the like (for example, diazo-based coloring reagents such as methyl orange, methyl red, congo red, and methyl yellow, and sultone-based coloring reagents such as thymol blue, bromocresol green, bromocresol purple, and bromothymol blue), which react with H⁺ ions and develops color and which are well-known coloring reagents, in accordance with the pH of the aqueous solution containing the amplification reagent. Among them, 1,10-phenanthroline, bathophenanthroline, or bromocresol green can be more preferably used.

The coloring reagent is preferably a coloring reagent that does not substantially move in the porous carrier in a case where any of an aqueous solution containing a test specimen or an aqueous solution containing the reducing agent for silver ions is spread. Accordingly, LogP (partition coefficient in water and octanol) of the coloring reagent is preferably 4.0 or more, and more preferably 5.0 or more. An actual measurement value may be used as LogP, but a calculation value obtained from a chemical structure or the like can also be used as a simple judging method. As a method of calculating LogP, a calculation method used in ChemDraw Pro version 12 of Cambridge Soft is preferable. Responsiveness and LogP (according to ChemDraw Pro version 12) of representative coloring reagents are shown in Table 1.

**[Table 1]**

| Compound name | Reactivity | LogP |
|---|---|---|
| 2,2'-Bipyridine | Fe²⁺ reaction | 1.88 |
| Bathophenanthrolinedisulfonic acid | Fe²⁺ reaction | 0.52 |
| 1,10-Phenanthroline | Fe²⁺ reaction | 2.2 |
| 5-Methyl phenanthroline | Fe²⁺ reaction | 2.69 |
| 5-Nitrophenanthroline | Fe²⁺ reaction | 2.34 |
| Thymol blue | pH reaction | 4.01 |
| Methyl orange | pH reaction | 2.95 |
| Methyl red | pH reaction | 3.63 |
| Congo red | pH reaction | 3.63 |
| Methyl yellow | pH reaction | 4.76 |
| Bathophenanthroline | Fe²⁺ reaction | 5.55 |
| Bromocresol green | pH reaction | 7.99 |
| Bromocresol purple | pH reaction | 6.33 |
| Bromothymol blue | pH reaction | 8.8 |

A region having the coloring reagent is preferably located downstream of the label substance-trapping region having the reaction site of the porous carrier. As a method of holding the coloring reagent in the chromatographic kit, there are a method of immersing a water absorption pad to be described later in a coloring reagent solution and drying under reduced pressure, a method of linearly applying in a downstream direction from a label substance-trapping region of a porous carrier, and the like.

In a case where the coloring reagent substantially moves in the porous carrier in a case where the aqueous solution containing the test specimen or the aqueous solution containing the second amplification reagent is spread, it is preferable that the coloring reagent be contained in the water absorption pad and used.

In a case where the coloring reagent does not substantially move in the porous carrier in a case where the aqueous solution containing the test specimen or the aqueous solution containing the second amplification reagent is spread, it is preferable to cause the porous carrier having the label substance-trapping region to carry the coloring reagent.

In the present invention, an aspect in which the coloring reagent is carried by the porous carrier is more preferable because then it is possible to display arrival of the second amplification reagent in the label substance-trapping region with a smaller time lag.

In the present invention, in a case where a region to which a test specimen containing a test substance is added, and a label substance-trapping region are provided in this order from an upstream direction to a downstream direction with respect to a spreading direction of the test specimen containing the test substance, an upstream direction and a downstream direction with respect to the spreading direction of the test specimen containing the test substance are defined in a case where the test specimen is spread by utilizing capillarity, suction power in a case where the water absorption pad is used, or the like. In a specific aspect of the present invention, in a case where a test specimen and the like are spread from the label substance-holding region toward the label substance-trapping region, a direction of the label substance-holding region is defined as an upstream direction, and a direction of the label substance-trapping region is defined as a downstream direction.

In a preferred aspect of the present invention, the second amplification reagent of the two kinds of amplification reagents used for amplifying signals of a label substance trapped in the label substance-trapping region is spread from the upstream direction of the label substance-trapping region to the downstream direction of the label substance-trapping region to detect physical or chemical changes in the region having the coloring reagent, and thereby it is possible to confirm that the label substance-trapping region is filled with the second amplification reagent. As the physical or chemical changes in the region having the coloring reagent, it is possible to detect changes in color development or fluorescence caused by a reaction between the second amplification reagent and the coloring reagent. Color development can be preferably detected. Such physical or chemical changes may be detected visually or may be detected using a detection device.

### 6. Method of inspection

A sandwich method and a competitive method which are specific embodiments for the chromatographic method of the embodiment of the present invention will be described below.

The sandwich method is not particularly limited, but for example, analysis of a test substance can be performed by the following procedure. First, the first antibody having binding properties with respect to the test substance, and the second antibody having binding properties with respect to the test substance are prepared in advance. In addition, label substances are modified with the first antibody in advance. In a case where the second antibody is immobilized on an appropriate chromatographic carrier (porous carrier) (for example, nitrocellulose membrane, glass fiber membrane, nylon membrane, cellulose membrane, and the like) so as to serve as a label substance-trapping region, and this region is brought into contact with a test specimen (or an extraction liquid thereof) that may contain a test substance, binding to the second antibody (for example, an antigen-antibody reaction with the second antibody) occurs in a case where the test substance is present in the test specimen. In a case where an excess amount of label substances modified with the first antibody is further brought into contact with the region at the same time of binding of the test substance and the second antibody, or after binding thereof, a composite body consisting of the immobilized second antibody, the test substance, and the label substances modified with the first antibody is formed in a case where the test substance is present in the test specimen.

In the sandwich method, it is possible to determine the presence or absence of the test substance in the test specimen or measure an amount thereof by removing a label substance that did not form an immune composite body after the reaction of the immobilized second antibody with the test substance, and the test substance with the first antibody with which the label substances are modified is completed, and thereafter, by, for example, observing a label substance-trapping region of a porous carrier as it is, and detecting or quantitatively determining a label substance. In the present invention, for example, a reducing agent and a silver ion-containing compound are supplied, and thereby signals from a label substance that has formed such a composite body are amplified and detected.

The competitive method is not particularly limited, but for example, analysis of a test substance can be performed by the following procedure. The competitive method is known as a technique of detecting an antigen of a low-molecular-weight compound that cannot be assayed by the sandwich method. First, the first antibody having specificity for the test substance (antigen) is prepared in advance. In addition, label substances are modified with the first antibody in advance. The test substance itself, which is a binding substance for the first antibody, or a compound which is similar to the test substance and has a site similar to that of the test substance, is immobilized on an appropriate binding substance-immobilized membrane (for example, nitrocellulose membrane, glass fiber membrane, nylon membrane, cellulose membrane, and the like) and used as a label substance-trapping region. In a case where a test specimen (or an extraction liquid thereof) that may contain the test substance (antigen), and a label substance modified with the first antibody in advance are brought into contact with each other, and in a case where the test substance is not present in the test specimen, a composite body is formed from the first antibody with which the label substance is modified, and the test substance itself, which is immobilized on the label substance-trapping region and has binding properties with respect to the first antibody, or the compound similar to the test substance. On the other hand, in a case where the test substance is present in the test specimen, because the test substance (antigen) binds to the first antibody with which the label substance is modified, binding to the test substance itself, which immobilized on the label substance-trapping region and has binding properties with respect to the first antibody, or the compound similar to the test substance is hindered, and therefore a composite body is not formed.

After the reaction between the test substance itself or the compound similar to the test substance having a site similar to that of the test substance, and the first antibody with which the label substance is modified is completed, the first antibody with which an unbound label substance is modified is removed. Subsequently, an amount of the label substance modified with the first antibody in the label substance-trapping region is detected or quantitatively determined, and thereby the presence or absence of the test substance in the test specimen can be determined, or an amount thereof can be measured. In the present invention, for example, the reducing agent for silver ions and the compound containing silver are supplied, and thereby signals from the label substance that has formed the composite body are amplified and detected.

### 7. Amplification reagent

An amplification reagent is a reagent that can cause signal amplification by catalytically reacting due to an action of a label substance or a test substance and causing coloration of a compound, luminescence, and the like. The amplification reagent can used in a state of a solution containing a reagent, that is, an amplification liquid. Examples thereof include a silver ion solution that causes metal silver to precipitate on a metal label by physical development, a solution of a phenylenediamine compound and a naphthol compound which serves as a colorant due to an action of a peroxidase label and hydrogen peroxide, and the like.

For details, a so-called developer can be used as an amplification liquid containing an amplification reagent, where developers are described in general books in the field of photographic chemistry (for example, "Fundamentals of Photograph Engineering (Revised) -Silver Halide Photography-" (edited by the Society of Photography and Imaging of Japan, Corona Publishing Co., Ltd.), "Chemistry of Photography" (by Akira SASAI, Shashinkogyo Publishing Company), and "Latest Prescription Handbook" (by Shinichi KIKUCHI et al., Amiko Publishing Company)). It is possible to use any developer as an amplification liquid without particular limitation as long as it is a so-called physical developer which contains silver ions in the liquid and in which the silver ions in the liquid are reduced mainly by metal colloids and the like which form the core of development.

In the present invention, two kinds of amplification reagents are used. It is preferable that a first amplification reagent be incorporated in a first amplification liquid, and a second amplification reagent be incorporated in a second amplification liquid among the two kinds of amplification reagents used for amplifying signals of label substances trapped in the label substance-trapping region, and amplification be performed by adding the first amplification liquid and the second amplification liquid in this order.

As a specific example of the amplification liquid, it is possible to use a combination of the first amplification liquid containing the compound containing silver, and the second amplification liquid containing the reducing agent for silver ions (reducing agent that can reduce silver ions).

Hereinafter, the compound containing silver contained in the first amplification liquid, and the reducing agent for silver ions contained in the second amplification liquid will be described.

### 7-1. Compound containing silver

As the compound containing silver, it is possible to use silver ion-containing compounds such as organic silver salts, inorganic silver salts, or silver complexes. The compound is preferably a silver ion-containing compound having a high solubility in solvents such as water, and examples thereof include silver nitrate, silver acetate, silver lactate, silver butyrate, silver thiosulfate, and the like. Silver nitrate is particularly preferred. Silver complexes are preferably silver complexes coordinated with ligands having a water-soluble group such as a hydroxyl group or a sulfone group, and examples thereof include silver hydroxythioether and the like.

In general, as silver, inorganic silver salts or silver complexes are contained in an amount of 0.001 mol/m² to 0.2 mol/m², and are preferably contained in an amount of 0.01 mol/m² to 0.05 mol/m².

### 7-2. Reducing agent for silver ions

As the reducing agent for silver ions, any inorganic or organic material or a mixture thereof can be used as long as it can reduce silver ions to silver.

Preferred examples of inorganic reducing agents include reducing metal salts and reducing metal complex salts which are capable of changing an atomic value with metal ions such as a divalent iron ion (Fe²⁺), V²⁺, or Ti³⁺. In a case in which an inorganic reducing agent is used, it is necessary to remove or detoxify oxidized ions by complexing or reducing the oxidized ions. For example, in a system in which Fe²⁺ is used as a reducing agent, a complex of Fe³⁺, which is an oxide, can be formed using citric acid or EDTA and detoxified. In the present system, such an inorganic reducing agent is preferably used, and a metal salt of Fe²⁺ is more preferred.

It is also possible to use a main developing agent used in a light-sensitive silver halide photographic material of a wet-type (such as methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), and leuco colorants), and other materials obvious to those who are skilled in the technology in the present field, such as a material disclosed in US6020117A.

As the reducing agent, an ascorbic acid reducing agent is also preferable. Useful ascorbic acid reducing agents include ascorbic acid and analogs thereof, and isomers and derivatives thereof. Preferred examples thereof include D- or L-ascorbic acid and sugar derivatives thereof (such as γ-lactoascorbic acid, glucoascorbic acid, fucoascorbic acid, glucoheptoascorbic acid, and maltoascorbic acid), a sodium salt of ascorbic acid, a potassium salt of ascorbic acid, isoascorbic acid (or L-erythroascorbic acid), a salt thereof (such as alkali metal salts, ammonium salts, or salts known in the technical field), ascorbic acid of the enediol type, ascorbic acid of the enaminol type, ascorbic acid of the thioenol type, and the like. Particularly preferred examples thereof include D-, L-, or D,L-ascorbic acid (or an alkali metal salt thereof) or isoascorbic acid (or an alkali metal salt thereof), and a sodium salt is a preferable salt. A mixture of these reducing agents can be used as necessary.

### 8. Other auxiliaries

As other auxiliaries of the amplification liquid, buffers, preservatives such as antioxidants or organic stabilizers, and rate regulators may be included. As the buffer, it is possible to use, for example, acetic acid, citric acid, sodium hydroxide, or salts of these substances, or a buffer formed of tris(hydroxymethyl)aminomethane or other buffers used in general chemical experiments. A pH can be adjusted to an optimum pH for the amplification liquid by appropriately using these buffers. Furthermore, an alkylamine can be used as an additive as an antifogging agent, and dodecylamine is particularly preferable. In addition, a surfactant can be used in order to improve solubility of these additives, and C₉H₁₉-C₆H₄-O-(CH₂CH₂O)₅₀H is particularly preferable.

As a method of spotting the amplification reagent on the chromatographic kit, a method is preferable in which a reducing agent solution as the second amplification liquid is spotted on a pad for liquid feeding of the reducing agent solution, a silver ion solution as the first amplification liquid is spotted from above on a region including a label substance-trapping region, and the silver ion solution is infiltrated in a thickness direction of a porous carrier.

As a method of incorporating the two kinds of amplification reagents into the chromatographic kit, there is a method of disposing a pot containing a solution containing each of the amplification reagents above a site at which each of the amplification reagents is spotted. It is preferable that the reducing agent solution (second amplification liquid) be placed on the pad for liquid feeding of the reducing agent solution, and the pot containing the silver ion solution (first amplification liquid) be placed immediately above a hole filled with the silver ion solution. By disposing in this manner, the liquid flows by pushing each pot and can be spotted on a predetermined site.

### 9. Chromatographic kit

In the chromatographic kit of the embodiment of the present invention, the label substance modified with the first antibody having binding properties with respect to the test substance may be provided on the porous carrier in advance, or alternatively, the label substance modified with the first antibody having binding properties with respect to the test substance may be provided separately from the porous carrier. In this case, the label substance modified with the first antibody having binding properties with respect to the test substance, which is provided separately from the porous carrier, can be measured by a method such as a method of mixing the label substance with a test specimen and then spreading the mixture on the porous carrier.

The chromatographic kit of the embodiment of the present invention further includes the compound containing silver and the reducing agent for silver ions.

The chromatographic kit of the embodiment of the present invention may include a housing case encompassing the porous carrier having a reaction site, the compound containing silver, and the reducing agent for silver ions.

The chromatographic kit of the embodiment of the present invention may further include pots each having a tearable member, in which the compound containing silver and the reducing agent for silver ions may be respectively sealed in the pots. In this case, the above-mentioned pots can be broken by an external force.

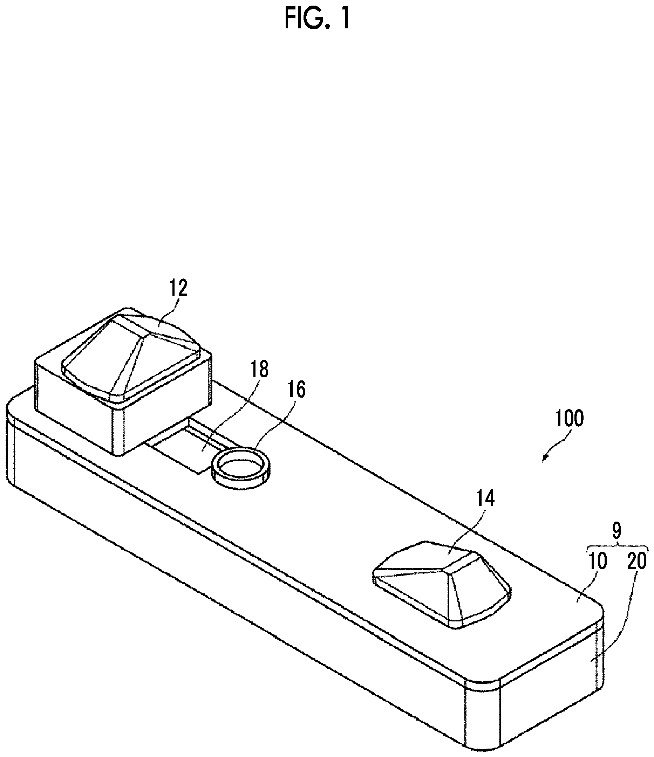

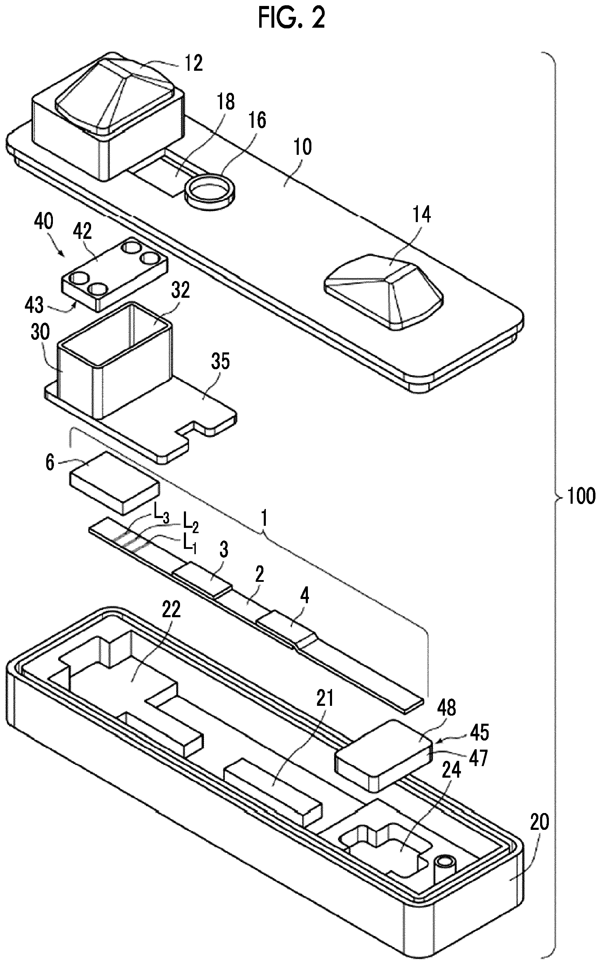

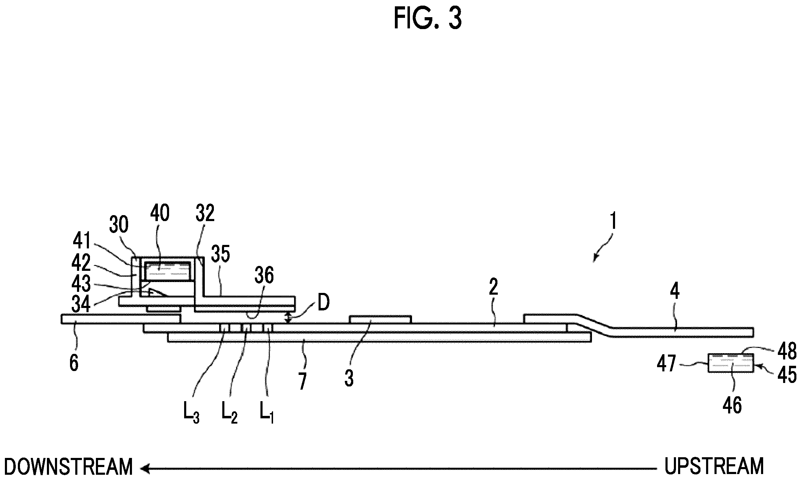

Fig. 1 is a schematic perspective view illustrating a chromatographic kit 100 showing an example of the present invention, and Fig. 2 is an exploded schematic perspective view of the chromatographic kit 100 of Fig. 1. Fig. 3 is a schematic side view showing a positional relationship between an inspection strip, and a first pot and a second pot.

As illustrated in Fig. 1 to Fig. 3, in the chromatographic kit 100 of the present embodiment, a housing case 9 encompasses an inspection strip 1 that has a porous carrier 2 having an inspection region of a test substance and is for spreading a specimen liquid, and a first pot 40 and a second pot 45 which are for amplifying a detection signal in the inspection region, which respectively include a surface having a sheet member, and in which a first amplification liquid 41 and a second amplification liquid 46 are sealed, respectively. The housing case 9 includes a lower case 20 having an accommodation portion 21 in which the inspection strip 1 is disposed, an upper case 10 joined to the lower case 20 along a peripheral edge, and a middle member 30 disposed between the upper case 10 and the lower case 20. In explaining the chromatographic kit 100 of the present embodiment, the upper case 10 side is defined as an upper part and the lower case 20 side is defined as a lower part.

The middle member 30 has a pot accommodation portion 32 which accommodates the first pot 40 and which has, on a bottom surface, an amplification liquid-filled hole for dropwise addition of the first amplification liquid 41 onto the porous carrier 2. In addition, a protrusive tearing portion 34 that tears up a sheet member 43 is provided at a location facing the sheet member 43 of the first pot 40 in the pot accommodation portion 32. In the present example, the first pot 40 is disposed above the pot accommodation portion 32 so that the surface having the sheet member 43 is the lower surface, and the tearing portion 34 is provided on the bottom surface of the pot accommodation portion 32 facing the sheet member 43 (refer to Fig. 3).

In addition, a flow path-forming portion 35 is provided so as to extend to a downstream side of the bottom surface of the pot accommodation portion 32 of the middle member 30. The flow path-forming portion 35 is disposed to correspond with positions above an inspection region L₁, a confirmation region L₂, and an amplification index region L₃, and is formed of a transparent material so that these regions L₁ to L₃ can be visually checked.

The upper case 10 includes, on a part facing the first pot 40, a first protrusive deforming portion 12 that is deformed towards the first pot 40 side so as to allow the tearing portion 34 of the middle member 30 to tear up the sheet member 43 of the first pot 40 by application of a pressing force from the outside. In addition, the upper case 10 includes, on a part facing the second pot 45, a second protrusive deforming portion 14 that is deformed towards the second pot 45 side so that a sheet member 48 of the second pot 45 is torn up by application of a pressing force from the outside.

In addition, an opening hole 16 for dropwise addition of a specimen liquid is provided on the upper case 10, and a specimen liquid is added dropwise onto a label-holding pad 3 of the inspection strip 1 from this opening hole 16. In a case where a location of the label-holding pad 3 is adjusted so that locations of the opening hole 16 and the label-holding pad 3 correspond to each other, it is possible to reliably spot the specimen liquid onto the label-holding pad 3. In addition, the upper case 10 includes an observation window 18 for visually checking the three regions L₁ to L₃ at positions corresponding to the flow path-forming portion 35 of the middle member 30.

In the lower case 20, as an accommodation portion in which the inspection strip 1 is disposed, a porous carrier accommodation portion 21 on which the porous carrier 2 is placed is provided, and an absorption pad accommodation portion 22 on which an absorption pad 6 is placed is provided on the downstream side of the porous carrier accommodation portion. In addition, a second pot accommodation portion 24 in which the second pot 45 is accommodated is provided on the upstream side of the porous carrier accommodation portion 21.

Fig. 3 is a schematic cross-sectional view illustrating a positional relationship between the inspection strip 1, the middle member 30, and the two pots 40 and 45. As illustrated in Fig. 3, the inspection strip 1 includes the porous carrier 2 spreading the specimen liquid, the label-holding pad 3 having a label substance modified with an antibody immobilized on the porous carrier 2, a liquid-feeding pad 4 which is disposed in contact with one end of the porous carrier 2 and feeds the second amplification liquid 46 to the porous carrier 2, and the absorption pad 6 disposed in contact with the other end of the porous carrier 2. The porous carrier 2 is immobilized to and supported by a back pressure-sensitive adhesion sheet 7. In addition, between the label-holding pad 3 and the absorption pad 6, the porous carrier 2 has the inspection region L₁, the confirmation region L₂, and the amplification index region L₃ in this order from the label-holding pad 3 side.

In the present specification, there are cases in which the porous carrier 2, which has the inspection region L₁, the confirmation region L₂, and the amplification index region L₃ formed thereon, is referred to as a chromatographic carrier. In addition, in the present specification, as illustrated in Fig. 3, the liquid-feeding pad 4 side is defined as an upstream side and the absorption pad 6 side is defined as a downstream side.

The middle member 30 is positioned at the upper portion and the downstream end side of the inspection strip 1, and the first pot 40 is disposed in the pot accommodation portion 32 of the middle member 30 with the sheet member 43 facing downward. The second pot 45 is accommodated at the lower portion and the upstream end of the inspection strip 1 in the lower case 20 with the sheet member 48 facing upward.

As shown in Fig. 3, a gap (clearance) D is formed between a rear surface 36 of the flow path-forming portion 35 of the middle member 30, and the porous carrier 2 of the inspection strip 1. The gap D is preferably within a range of 0.01 mm to 1 mm. In a case where the gap is 0.01 mm or more, an amplification liquid and the like can be sufficiently infiltrated, and in a case where the gap is 1 mm or less, capillary force is exerted and the gap between the porous carrier 2 and the middle member 30 can be uniformly filled with the first amplification liquid 41.

In the first pot 40 in which the first amplification liquid 41 is sealed, for example, a container 42 which is formed of a resin material and has an opening on one surface is filled with the first amplification liquid 41, and the opening of the container 42 is covered with the tearable sheet member 43 and sealed.

Similarly, in the second pot 45 in which the second amplification liquid 46 is sealed, for example, a container 47 which is formed of a resin material and has an opening on one surface is filled with the second amplification liquid 46, and the opening of the container 47 is covered with the tearable sheet member 48 and sealed.

As the tearable sheet members 43 and 48 in the first pot 40 and the second pot 45, laminate films such as aluminum foils and aluminum laminate sheets are suitability used. The term "tear" refers to a state in which the sheet does not regenerate after being torn up.

### 10. Method of calculating average particle size of label substance in detection

In detection (after amplification), a test line portion is cut out, a rear surface of a specimen is attached to a specimen stand with carbon paste, and thereafter, a cross section is cut, carbon vapor deposition is performed, and a shape and a size are observed with a scanning electron microscope. For example, observation of a specimen surface by reflected electrons at an accelerating voltage of 10 KV using a FE-STEM S-5500 manufactured by Hitachi High-Technologies Corporation can be performed with a scanning electron microscope (SEM). Thereafter, 100 signal particles are selected, a circle equivalent diameter of a projection area of the particles is measured, and an average value is calculated and used for an average particle size in the detection.

An average particle size of the label substance in the detection is preferably 1 µm or more and 20 µm or less, and more preferably 3 µm or more and 20 µm or less.

Hereinafter, the present invention will be described more specifically with reference to Examples of the present invention. In the following Examples, materials, amounts used, ratios, details of a treatment, treatment procedures, and the like may be appropriately changed without departing from the gist of the present invention. Accordingly, the scope of the present invention should not be limitedly interpreted by the following specific examples. Examples

Immunochromatographic kits of an example and a comparative example are NS1 antigens-detecting immunochromatographic kits which are for detecting dengue virus NS1 antigens as a test substance. The acquisition of a monoclonal antibody used in the kit is described below.

### [Production of anti-NS1 mouse monoclonal antibody]

Mice were administered a total of 3 times with an emulsion in which 100 µg of a sensitizing antigen (NS1) was mixed with a complete Freund's adjuvant (FCA). Thereafter, blood was collected to measure the antibody valence, and iliac lymph nodes (spleen) were collected from mice in which antibody activity was confirmed to prepare lymphocytes. Thereafter, cell fusion with myeloma was performed, and cloning was performed by a limiting dilution method to obtain hybridomas of each clone. Thereafter, the hybridomas of the two clones, which were highly reactive by an Enzyme-Linked Immunosorbent Assay (ELISA) method, were expansion-cultured in a GIT (registered trademark) medium (manufactured by FUJIFILM Wako Pure Chemical Corporation), and the culture supernatant was purified on a Protein A column to obtain two types of anti-NS 1 mouse monoclonal antibodies. One of the two types thereof was converted to F(ab')₂ as an anti-NS1 mouse monoclonal antibody 1 by the method described below. The other of the two types was designated as an anti-NS1 mouse monoclonal antibody 2 and used as an antibody immobilized on a nitrocellulose membrane as described below.

### [Production of F(ab')₂ anti-NS1 mouse monoclonal antibody]

For the anti-NS1 mouse monoclonal antibody, an F(ab')₂ anti-NS1 mouse monoclonal antibody was produced using a Pierce (TM) Mouse IgG1 Fab and F(ab')₂ Preparation Kit (product No. 44980, Thermo Fisher Scientific).

### (1) Production of immunochromatographic kit

### (1-1) Production of F(ab')₂ anti-NS1 mouse monoclonal antibody-modified gold colloid as label substance modified with first antibody capable of binding to test substance

The pH was adjusted by adding 1 mL of 50 mmol/L KH₂PO₄ buffer (pH 8.0) to 9 mL of a solution (product No.: EM.GC50, manufactured by BBI) containing gold colloid with the diameter of 50 nm. Thereafter, 1 mL of a solution containing 20 µg/mL of the F(ab')₂ anti-NS1 mouse monoclonal antibody produced as described above was added, and the mixture was stirred for 10 minutes. Thereafter, after the solution mixture was left to stand for ten minutes, 550 µL of an aqueous solution containing 1 mass% polyethylene glycol (PEG; weight-average molecular weight (Mw.): 20,000, product No. 168-11285, manufactured by FUJIFII,M Wako Pure Chemical Corporation) was added to the solution mixture and stirred for 10 minutes. Subsequently, 1.1 mL of an aqueous solution of 10 mass% bovine serum albumin (BSA; Fraction V, product No.: A-7906, manufactured by Sigma-Aldrich Co. LLC.) was added thereto and stirred for 10 minutes. This solution was centrifuged for 30 minutes under conditions of 8,000 × g at 4°C using a centrifugal separator (himacCF16RX, manufactured by Hitachi Ltd.). The supernatant liquid was removed with 1 mL thereof remaining at the bottom of a container, and gold colloid contained in the 1 mL solution remaining at the bottom of the container was re-dispersed by an ultrasonic washer. Thereafter, the solution was dispersed in 20 mL of a gold colloid preservative solution (20 mmol/L Tris-HCl (tris hydrochloric acid) buffer (pH 8.2), 0.05% PEG (molecular weight: 20,000), 150 mmol/L NaCl, 1% BSA), and again, centrifugation was performed under the same conditions using the same centrifugal separator to remove the supernatant liquid and perform ultrasonic dispersion. Thereafter, the solution was dispersed in the gold colloid preservative solution to obtain an antibody-modified gold colloid (50 nm) solution.

### (1-2) Production of anti - NS 1 antibody-modified gold colloid-holding pad as label-holding pad

The F(ab')₂ anti-NS1 mouse monoclonal antibody-modified gold colloid produced in (1-1) was diluted with water so that a concentration of a Tris-HCl buffer (pH: 8.2) reached 20 mmol/L, a concentration of PEG (molecular weight: 20,000) reached 0.05 mass%, a concentration of sucrose reached 5 mass%, and an optical density of the gold colloid at 520 nm reached 0.1 in a case where an optical path length was set to 10 mm, and thereby a gold colloid coating liquid was obtained. This coating liquid was uniformly applied onto glass fiber pads each cut into 5 mm × 300 mm (Glass Fiber Conjugate Pad, manufactured by EMD Millipore Corporation) by 1 mL per pad, and was dried at reduced pressure for 24 hours. Thereby an F(ab')₂ anti-NS1 mouse monoclonal antibody-modified gold colloid-holding pad was obtained.

### (1-3) Production of chromatographic carrier

Using nitrocellulose membrane cut into 60 mm × 300 mm (with a plastic backing, HiFlow Plus HF135 (capillary flow rate = 135 sec/cm), manufactured by EMD Millipore Corporation) as a porous carrier, an inspection region, a confirmation region, and an amplification index region were formed on this membrane by a method described below, and thereby a chromatographic carrier was produced.

An aqueous solution of the anti-NS1 mouse monoclonal antibody 2, which was prepared so that the concentration was 1.5 mg/mL, was applied in a line shape at a position 15 mm from the downstream side of a 60 mm short side of the nitrocellulose membrane to define the inspection region. Furthermore, an anti-mouse IgG rabbit polyclonal antibody (manufactured by Jackson) solution, which was prepared so that the concentration was 0.5 mg/mL, was applied in a line shape at a position 11 mm from the downstream side of the 60 mm short side to define the confirmation region. Furthermore, a Bromocresol Green (manufactured by FUJIFILM Wako Pure Chemical Corporation), which was prepared so that a concentration was 30 mmol/L, was applied in a line shape at a position 9 mm from the downstream side of the 60 mm short side to define the amplification index region. After the application of the respective solutions, the nitrocellulose membrane was dried at 50°C for 30 minutes using a warm air-type dryer. After completion of the drying, the nitrocellulose membrane dried as described above was immersed in a vat in which 500 mL of a blocking liquid (50 mmol/L of a boric acid buffer (pH: 8.5) containing 0.5 mass% casein (derived from milk, product No. 030-01505, manufactured by FUJIFILM Wako Pure Chemical Corporation)) was put, and the membrane was left to stand for 30 minutes. Thereafter, the nitrocellulose membrane was taken out, the nitrocellulose membrane was immersed in 500 mL of a washing and stabilizing liquid (50 mmol/L Tris-HCl (pH: 7.5) buffer containing 0.5 mass% sucrose and 0.05 mass% sodium cholate) prepared in another vat, and the membrane was left to stand for 30 minutes. Thereafter, the nitrocellulose membrane was removed from the liquid and dried at an environment of 25°C for 24 hours.

A part to which the anti-NS1 mouse monoclonal antibody 2 was immobilized corresponded to the inspection region (reaction site) including the second antibody that binds to the test substance, a part to which the anti-mouse IgG rabbit polyclonal antibody was immobilized corresponded to the confirmation region including the substance that can bind to the first substance, and a part to which the bromocresol green was immobilized corresponded to the amplification index region including the substance reacting with the first amplification liquid.

### (1-4) Production of inspection strip

The chromatographic carrier produced in (1-3) was attached to a back pressure-sensitive adhesion sheet (60 mm × 300 mm (manufactured by Adhesives Research)). Next, a 3 mm wide double-sided tape (NITTO DENKO CORPORATION) was fixed at a position 26 mm from the downstream side of a short side of the chromatographic carrier. Thereafter, the gold colloid-holding pad was fixed to the chromatographic carrier so that the downstream end of the double-sided tape and the downstream end of the anti-NS 1 antibody-modified gold colloid-holding pad prepared above overlapped each other. A liquid-feeding pad (glass fiber pad cut into 25 mm × 300 mm (Glass Fiber Conjugate Pad, manufactured by EMD Millipore Corporation) was attached to the upstream side of the chromatographic carrier so that the liquid-feeding pad and the chromatographic carrier overlapped each other by 7 mm. Using a guillotine style cutter (CM4000, manufactured by NTC/NIPPN TechnoCluster, Inc.)), a member produced in this manner was cut parallel to a direction perpendicular to a 300 mm long side such that a width was 5 mm. Thereby, 60 inspection strips (however, no absorption pads were included) were produced.

### (1-5) Production of amplification liquid

### (1-5-1) Production of amplification liquid (reducing agent solution) sealed in second pot

23.6 mL of an aqueous solution of 1 mol/L iron nitrate produced by dissolving iron (III) nitrate nonahydrate (manufactured by FUJIFILM Wako Pure Chemical Corporation, 095-00995) in water, and 13.1 g of citric acid (manufactured by FUJIFILM Wako Pure Chemical Corporation, 038-06925) were dissolved in 290 g of water. After the substances were fully dissolved, 36 mL of a nitric acid (10 wt%) solution was added thereto while stirring the solution using a stirrer, and 60.8 g of iron (II) ammonium sulfate hexahydrate (manufactured by FUJIFILM Wako Pure Chemical Corporation, 091-00855) was added thereto. The solution thus prepared was used for a reducing agent solution which was the second amplification liquid sealed in the second pot.

### (1-5-2) Production of amplification liquid (silver ion solution) sealed in first pot

8 mL of a silver nitrate solution (including 10 g of silver nitrate) and 24 mL of an aqueous solution of 1 mol/L iron nitrate were added to 66 g of water. Furthermore, this solution was mixed with a solution obtained by dissolving 5.9 mL of nitric acid (10 wt%), 0.1 g of dodecylamine (manufactured by FUJIFILM Wako Pure Chemical Corporation, 123-00246), and 0.1 g of a surfactant C₁₂H₂₅-C₆H₄-O-(CH₂CH₂O)₅₀OH in 47.6 g of water in advance. This mixed solution was used for a silver ion solution which was the first amplification liquid sealed in the first pot.

### (1-6) Production of absorption pad

60 glass fiber pads each cut into 12 mm × 10 mm (glass filter paper, manufactured by Advantech Co., Ltd.) were prepared and used for absorption pads.

### (1-7) Production of components of immunochromatographic kit

The lower case 20, the upper case 10, the middle member 30, the first pot 40, and the second pot 45 constituting the immunochromatographic kit 100 shown in Figs. 1 to 3 were respectively produced by injection molding using polypropylene as a material. The upper case was produced by injection molding using, as a material, polypropylene containing 50 mass% of TAFTHREN (registered trademark) which is an olefinic elastomer manufactured by Sumitomo Chemical Co., Ltd. The upper case 10 has two deformable portions (first protrusive deforming portion and second protrusive deforming portion). These two deforming portions are parts not separated from the upper case 10, and the upper case was produced by injection molding with the entire boundary as a part of the upper case 10.

As the configuration of the upper case of the example, the first protrusive deforming portion 12 shown in Figs. 1 and 2 has two protrusion portions, and the second protrusive deforming portion 14 has one protrusion portion.

### (1-8) Production of immunochromatographic kit of example

The lower case 20, the inspection strip produced in (1-4), and the absorption pad 6 produced in (1-6) were fixed as illustrated in Figs. 1 to 3. Next, the first pot 40 and the second pot 45 were respectively filled with the amplification liquid 41 to be sealed in the first pot 40 produced in (1-5-2) and the amplification liquid 46 to be sealed in the second pot 45 produced in (1-5-1). The pot 45 was sealed with aluminum foil as the sheet member 48, and the pot 40 was sealed with aluminum foil as the sheet member 43. As shown in Figs. 1 to 3, the second pot 45 was attached to the lower case 20 with the sheet member 48 facing upward, and the first pot 40 was attached to the middle member 30 with the sheet member 43 facing downward. In addition, in a state in which the upper case 10 and the lower case 20 were fitted so that the outer peripheries thereof came in contact with each other, contact portions of the upper case and the lower case were joined by ultrasonic welding. In this case, it was confirmed that the entire portions of the welded portions were uniformly welded in a sealed state. The immunochromatographic kit was produced in this manner.

### [Production of anti-CRP (C-reactive protein) mouse monoclonal antibody]

Mice were administered a total of 3 times with an emulsion in which a sensitizing antigen (CRP) was mixed with a complete Freund's adjuvant (FCA). Thereafter, blood was collected to measure the antibody valence, and spleen cells were collected from mice in which antibody activity was confirmed and mixed with myeloma cells to perform cell fusion. Thereafter, cell fusion with myeloma was performed, and cloning was performed by a limiting dilution method to obtain hybridomas of each clone. Thereafter, for the clones that were highly reactive by the ELISA method, an antibody was produced by a mouse ascites method and purified by a Protein A column to obtain an anti-CRP mouse monoclonal antibody to be used in the next [Production of particles modified with dummy antibody].

### [Production of particles modified with dummy antibody]

Latex particles were modified with the anti-CRP mouse monoclonal antibody with 250 mmol/L MES (pH 5.4 to 5.8).

As a preparation before starting the operation, the latex particles are irradiated with ultrasonic waves. Subsequently, mixing of the particles was performed. 96 µL of 250 mmol/L MES (pH 5.4 to 5.8), 25 µL of ultrapure water, and 300 µL of 2% latex particles were mixed.

### [Preparation of sample solution]

A diluent solution containing 0.10% by weight casein (030-01505, manufactured by FUJIFILM Wako Pure Chemical Corporation) and 0.1% by weight Tween (registered trademark) 80 was created. As a dummy antibody (that is, a third antibody having the same immune host as at least one antibody of the first antibody or the second antibody and not recognizing the test substance), an anti-CRP antibody derived from mice was used. The anti-CRP antibody derived from mice was modified into latex particles and added to the diluent solution as dummy particles. The level of the diluent solution was created using the latex particle concentration in the diluent solution as a parameter.

### [Evaluation of sample solution]

For the evaluation of the sample, amplification evaluation using an immunochromatographic kit was performed. 40 µL of the above-mentioned diluent solution was spotted to perform amplification evaluation.

In the amplification evaluation with the kit, the cases with a positive determination in the inspection region applied in the line shape were marked with +, and the cases with a negative determination were marked with -. In addition, the results of evaluation that were not carried out are described as NT.

### [Result 1] Result of evaluation (with amplification)

**[Table 2]**

| | Dummy antibody concentration [mg/mL] | Serum (no NS1) | NS1 (type 1) (8 ng/mL) |
|---|---|---|---|
| Comparative Example 1 | 0 | + | + |
| Example 1 | 0.0096 | - | + |
| Example 2 | 0.096 | - | NT |
| Example 3 | 0.191 | - | NT |
| Example 4 | 0.239 | - | + |
| Example 5 | 0.382 | - | NT |
| Example 6 | 0.478 | - | + |

### · Comparative Example 2

### [Result 2] Results of evaluation (without amplification) using commercially available kit (Abbott, Bioline Dengue DUO NS1AG + IGG/IGM)

**[Table 3]**

| Serum (no NS1) | NS1 (type 1, 8 ng/mL) | NS1 (type 1, 100 ng/mL) |
|---|---|---|
| - | - | + |

Based on the result 1 and the result 2, the commercial product (without amplification) was low-sensitivity because positive results were obtained for 100 ng/mL NS1, negative results were obtained for serum (without NS1) 8, and negative results were obtained for 8 ng/mL NS1.

In the case where silver amplification was performed (result 1), high-sensitivity was shown as NS1 of 8 ng/mL became positive, but because of the high-sensitivity, false positives occurred even with a small amount of non-specific adsorption in serum. However, in a case where 0.0096 mg/mL or more of the antibody having the same immune host as that of the first antibody having binding properties with respect to the test substance was added, false positives in serum (without NS1) could be inhibited while maintaining high-sensitivity.

### [Result 3] False positives depending on sample species

**[Table 4]**

| | Antibody concentration [mg/mL] | Serum ① (no NS1) | Serum ② (no NS1) | Runny nose ① (no NS1) | Runny nose ② (no NS1) |
|---|---|---|---|---|---|
| Comparative Example 3 | 0 | + | + | - | - |
| Example 7 | 0.096 | - | - | - | - |

False positives did not occur in the runny nose which had less impurities as compared to serum. False positives could be inhibited by adding 0.096 mg/mL of the antibody having the same immune host as that of the first antibody having binding properties with respect to the test substance to the serum containing a large amount of impurities.

### Explanation of References

1: inspection strip
2: porous carrier
3: label-holding pad (glass fiber pad)
4: liquid-feeding pad
6: absorption pad
7: back pressure-sensitive adhesion sheet
9: housing case
10: upper case
12: first protrusive deforming portion
14: second protrusive deforming portion
16: opening hole for dropwise addition of specimen liquid
18: observation window
20: lower case
21: porous carrier accommodation portion
22: absorption pad accommodation portion
24: second pot accommodation portion
30: middle member
32: first pot accommodation portion
34: tearing portion
35: flow path-forming portion
36: rear surface of flow path-forming portion 35
40: first pot for first amplification liquid
41: first amplification liquid
42: container
43: sheet member
45: second pot for second amplification liquid
46: second amplification liquid
47: container
48: sheet member
100: chromatographic kit
L₁: inspection region
L₂: confirmation region
L₃: amplification index region
D: gap (clearance)

## Claims

1. An immunochromatographic kit comprising:
a label substance modified with a first antibody having binding properties with respect to a test substance;
a porous carrier having a reaction site holding a second antibody having binding properties with respect to the test substance;
an amplification reagent; and
a third antibody having an immune host which is the same as that of at least one antibody of the first antibody or the second antibody and not recognizing the test substance.

2. The immunochromatographic kit according to claim 1, wherein the porous carrier further has a fourth antibody having binding properties with respect to the first antibody.

3. The immunochromatographic kit according to claim 1 or 2, wherein the third antibody is used at a concentration of 0.0096 mg/mL or more.

4. The immunochromatographic kit according to any one of claims 1 to 3, wherein at least one of the first antibody or the second antibody is F(ab')₂, Fab, Fab', or Fv.

5. The immunochromatographic kit according to any one of claims 1 to 4, wherein the label substance is a metal colloid.

6. The immunochromatographic kit according to any one of claims 1 to 5, wherein the amplification reagent contains a reducing agent for silver ions, and a compound containing silver.

7. The immunochromatographic kit according to claim 6, wherein the reducing agent for silver ions contains divalent iron ions.

8. The immunochromatographic kit according to any one of claims 1 to 7, wherein the immunochromatographic kit is used in measurement of the test substance in whole blood, serum, or plasma.

9. An immunochromatographic method comprising:
a step of spreading, on a porous carrier having a reaction site holding a second antibody having binding properties with respect to a test substance, the test substance, a label substance modified with a first antibody having binding properties with respect to the test substance, and a third antibody having an immune host which is the same as that of at least one antibody of the first antibody or the second antibody and not recognizing the test substance, in a state in which a composite body of the test substance and the label substance is formed;
a step of trapping the composite body at the reaction site; and
a step of amplifying a signal of the label substance with an amplification reagent to detect the test substance.

10. The immunochromatographic method according to claim 9, wherein the test substance is a test substance in whole blood, serum, or plasma.
